# EUROPEAN PATENT APPLICATION

(11) **EP 3 251 587 A1**
(43) Date of publication of application: **06.12.2017**
(21) Application number: 17000518.5
(22) Date of filing: 29.03.2017
(51) Int. Cl.: A61B 5/00

(54) **ECG MONITORING SYSTEM**

(30) Priority: 30.05.2016 PL 41735016
(71) Applicant: Comarch Healthcare Spólka Akcyjna, 31-864 Kraków (PL)
(72) Inventor: Przedzinski, Tomasz, 31-864 Kraków (PL); Kaleta, Lukasz, 31-864 Kraków (PL); Turek, Marcin, 31-864 Kraków (PL)

(57) **Abstract**

The subject of the invention is an ECG monitoring system consisting of: at least two electrodes (3) placed in an ECG examination garment (2), an ECG signal recorder (1) and a docking station (4). The recorder (1) is mounted on the garment (2) in a detachable manner. The detachable mount of the recorder (1) to the garment (2) consists of contacts (9) placed on the garment (2) in a number corresponding to the number of electrodes (3) and contacts (11) placed on the recorder (1). Each contact (9) is connected to an electrode (3). The recorder (1) is equipped with a switch (22) changing the recorder's work mode from saving signals from electrodes (3) to sending data to the docking station (4) and charging the recorder (1)

## Description

The subject of the invention is an ECG monitoring system designed to examine the electrical activity of the heart during a patient's everyday physical activities. The invention is a telemedical solution used to conduct the examinations by the patient themselves.

The traditional method of performing ECG examinations involves conducting measurements of the heart's electrical activity in a doctor's office. To do that, a patient must visit a doctor, who will conduct the examination and interpret the results. In that case, the patient will receive the results of their examination immediately. The drawback of this method of conducting ECG examinations is the fact that the patient must see a doctor personally. Additionally, such an examination only monitors a brief period of the heart's electrical activity within the entire day of the patient's life. Therefore, telemedical solutions started appearing as an answer to the drawbacks of ECG examinations performed in doctors' offices. Solutions of this type enable patients to conduct measurements of their heart's electrical activity personally, without the need to visit a doctor. Moreover, such solutions allow the examinations to be conducted at any time of day, during the patients daily physical activities.

International patent application WO 2010038156 describes a wireless ECG monitoring system. The ECG monitoring system includes a battery powering the ECG monitoring device and an adaptor receiving electrical signals from electrodes attached to a patient. Additionally, the ECG monitoring device contains a transmitter for wireless ECG data transmission and a circuit informing about the status, connected to the transmitter, used to send status updates when the status of the ECG monitoring device changes. The monitoring system also includes a cellular telephone, a receiver for wireless reception of ECG signals or status updates from the ECG monitoring device, and a driver controlling the operation of the cellular telephone, using it to send ECG data from the monitoring device to a monitoring centre through a cellular network. The driver can also respond to status updates from the ECG monitoring device by alerting the patient to status updates or by sending a status update to a monitoring centre.

The US patent description US 6416471 describes a solution involving a system and method of monitoring a patient's vital signs and remote collection of their data using radiotelemedical technologies. The system includes an array of disposable, wireless sensors measuring the full range of ECG waves, respiratory action, skin temperature, motion. The small unit (device) sending the signals can be worn by the patient, e.g. on their belt. The device transmits signals from the sensor array to a base station up to 60 metres away. The base station receiving the data from the device sending the signals is designed to be connected to a conventional telephone landline. The base station can also collect additional clinical data, such as blood pressure. The patient's safety is increased by the base station's ability to compare clinical data, e.g. ECG, with a preset profile and signal when the data is abnormal. The monitoring station allows presentation and viewing of the data sent by the sensor array. The ECG analysis software and user interface ensure remote analysis of the transmitted data. The solution has a useful application for collecting patients' clinical data during medication administration and medical examinations.

The European patent application EP 1815788 describes a solution involving a combination of a connecting mechanism, connector, electrical device, garment and physiological sensor. The connecting mechanism contains the first connecting part, which contains the first electrical conductive layer and first magnetic layer, and the second connecting part, which contains the second electrical conductive layer and second magnetic layer. The first and second magnetic layers provide a mechanical connection between the first connecting part and second connecting part, based on magnetic attraction between the first and second magnetic layer. The first electrical conductive layer and second electrical conductive layer provide an electrical connection between the first connecting part and second connecting part, based on magnetic attraction. In one of the embodiments of the solution, the connecting mechanism is used to provide a mechanical and electrical connection between the heartrate transmitter and the physiological sensor. In this event, the first connecting part is part of the heartrate transmitter while the second connecting part is part of the heartrate sensor. In this embodiment, the connecting mechanism enables fast and simple connecting and disconnecting of the transmitter and the sensor. In an example of use in clothing, the connecting mechanism can be used to ensure a mechanical and electrical connection between electrodes placed on the user and the heartrate transmitter. The electrodes can be separate or integrated with the clothing. The first connecting part can be connected with the electrode wire while the second connecting part can be connected to the heartrate transmitter.

The technical problem to be solved is how to create a system designed for ECG examinations, which would make ECG measurements easy enough for a patient to conduct the examination themselves, without the necessity to use qualified medical personnel. The goal of the invention is also proposing an ECG monitoring system which would be inexpensive to produce, making it widely available for individual persons. These goals have been achieved by the implementation of technical features used in the presented invention.

The subject of this solution is an ECG monitoring system consisting of: at least two electrodes placed in an ECG examination garment, an ECG signal recorder and a docking station. The recorder is mounted on the garment in a detachable manner and the docking station is equipped with communication measures for sending data to a server The invention characterized in that the detachable mount of the recorder to the garment consists of contacts placed on the garment in a number corresponding to the number of electrodes and contacts placed on the recorder, whose number and placement corresponds to the number and placement of the contacts. Each contact is connected to an electrode. Moreover, the recorder is equipped with a switch changing the recorder's work mode from saving signals from electrodes to sending data to the docking station and charging the recorder.

Preferably, the contacts in the garment and the contacts in the recorder create an electrical connection between the electrodes and the recorder. Each contact on the recorder is a rectangular metal strip with a central, rectangular indentation with a magnet installed, while the contacts are attached to the bottom wall of the recorder casing. Each contact of the garment is a rectangular metal strip. The circuit switching the recorder's work mode is connected to a set of reed switches placed in the recorder, each of which is assigned to a different contact. The recorder is equipped with a user interface, consisting of a button, an RGB diode and a buzzer. The docking station is a freestanding device connected to an external power source, equipped with a set of contacts corresponding to the number and placement of contacts on the recorder, with each contact being a rectangular metal strip with a pogo pin, installed directly under the upper wall of the docking station's casing. The docking station is equipped with a circuit for recharging the recorder. The docking station is equipped with a power switch containing a reed switch. The docking station is equipped with a user interface consisting of a button and three diodes. The garment is a vest consisting of a belt connected to suspenders. The electrodes are mounted on the vest belt. The electrodes are fabric electrodes. All the connectors are connected to electrodes with electric wires or conductive thread or fabric.

The advantage of the ECG monitoring system according to the invention is a solution which - thanks to its simplicity - allows patients to conduct ECG measurements personally, without the need to use the help of qualified medical personnel. The patient's role in conducting the ECG examination is limited to registering the ECG signals and sending the results out for analysis. A big advantage of the invention is the system's ease of operation, resulting from the construction of the individual elements making up the solution. Using a special garment to conduct the measurements ensures the proper placement of electrodes collecting the signal, which significantly reduces the risk of obtaining invalid results caused by improper electrode placement or loss of contact with the patient's body. The garment can be worn for extended periods of time, during everyday activities. This allows results to be gathered over long periods. Another advantage of the invention is the method used to attach the recorder to the garment. The magnet-equipped contacts provide both mechanical and electrical connection. This type of connection does not require great precision when the patient attaches the recorder. Another advantage of the solution is the low manufacturing cost and automation of the recorder charging process and data transfer to the docking station. Thanks to the use of reed switches, the recorder automatically switches its work mode from collecting signals from electrodes to sending the collected data to the docking station.

Embodiment of the invention is shown on drawings, where:
Fig. 1 shows the general scheme of the ECG monitoring system;
Fig. 2 shows an axonometric view of the docking station;

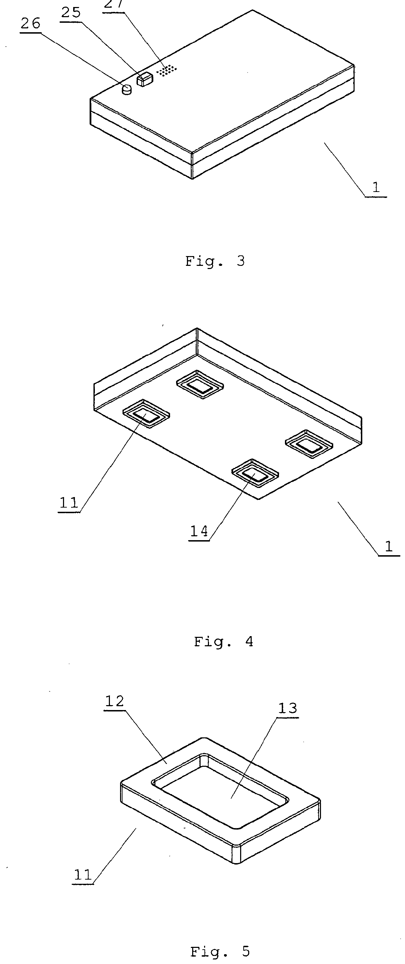

Fig. 3 shows an axonometric view of the recorder;
Fig. 4 shows the bottom view of the recorder;
Fig. 5 shows an axonometric view of a recorder contact;

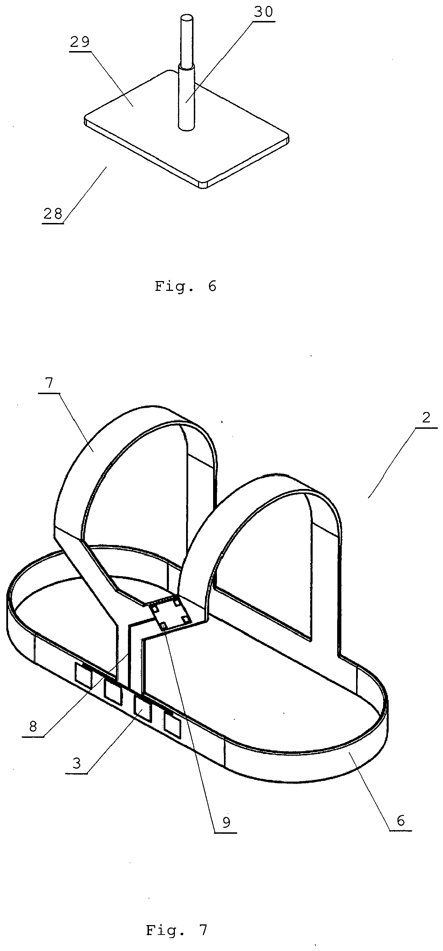

Fig. 6 shows an axonometric view of a docking station contact, without the magnet mounted in the opening;
Fig. 7 shows an axonometric view of the vest;

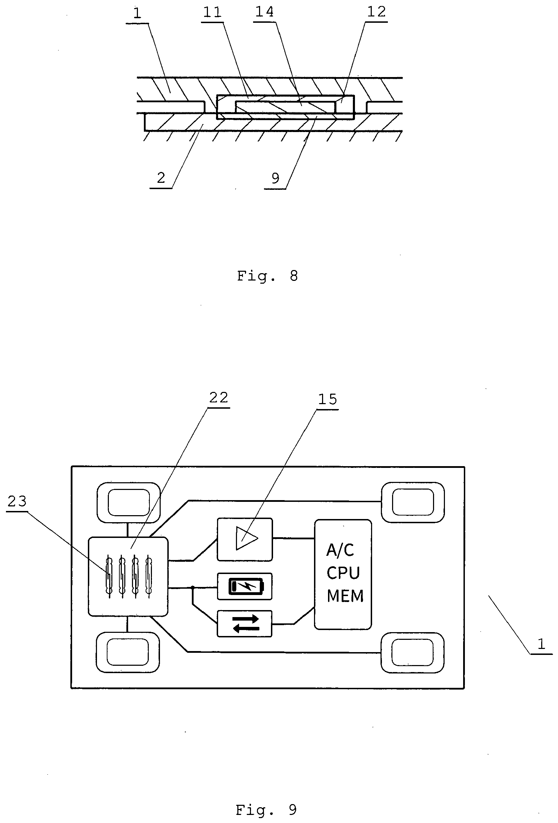

Fig. 8 shows a cross-section of the connection between a recorder contact with a vest contact;
Fig. 9 shows a scheme for an analog converter, the work mode switch circuit and reed switches installed in the recorder;

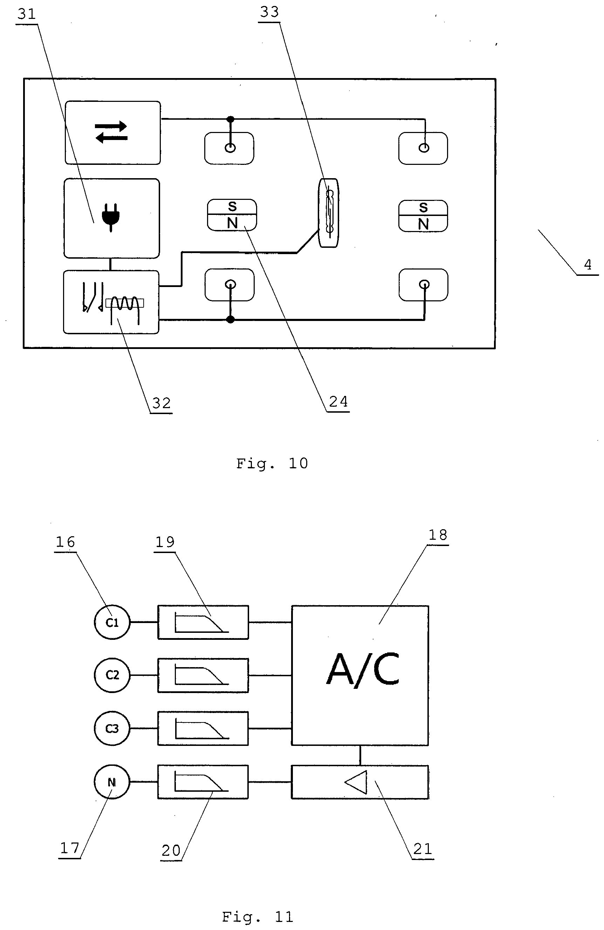

Fig. 10 is a scheme of reed switch and magnet placement in the docking station;
Fig. 11 shows a scheme of the analog converter in the recorder.

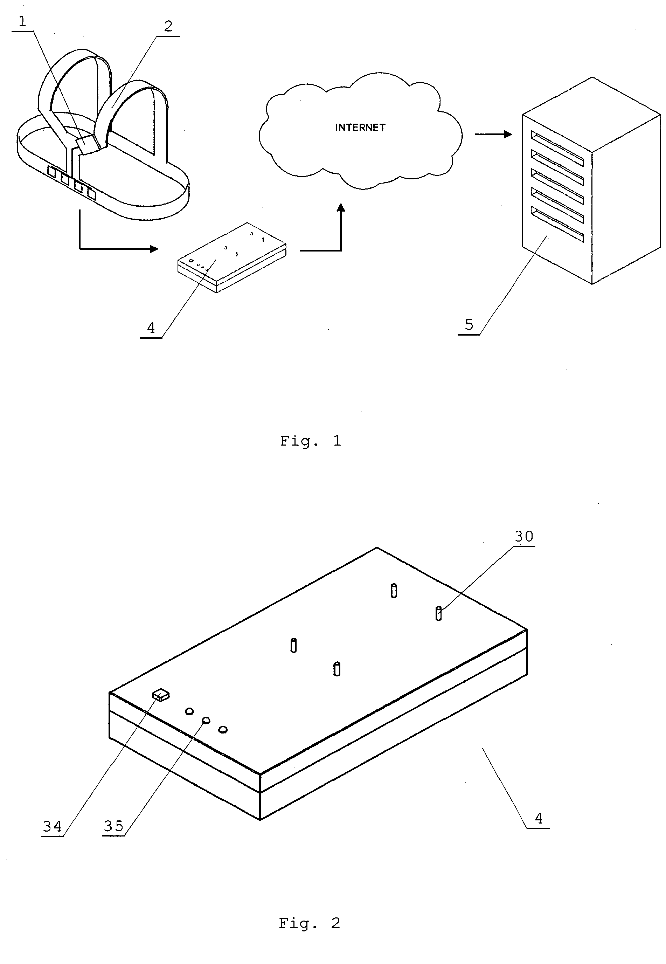

An embodiment of the ECG monitoring system has been presented as a general scheme on Fig 1. The system consists of the recorder 1, garment 2 designed specifically for ECG examinations, to which electrodes 3 have been attached, and of a docking station 4. To conduct an ECG examination, the patient will put on the vest and attach the recorder 1, which will record signals representing the electrical activity of the heart collected by the electrodes 3. Once the data has been collected, the patient will detach the recorder 1 from the vest 2 and connect it to the docking station 4, which will download the data from the recorder 1 and send them out for analysis. The docking station 4 will send the data to a server 5, where it will be processed, interpreted and shared with authorized persons. In the embodiment, the docking station 4 sends the data received from the recorder 1 to the server 5 via the Internet. In another possible embodiment, the data could be sent using a GSM network. Thanks to remote data analysis, the ECG monitoring system is a telemedical solution.

The garment is a vest, consisting of a belt 6 connected to suspenders 7. The belt 6, to which the suspenders 7 are connected, will pass right under the breast once the patient puts it on. The vest is made of fabric which ensures good adhesion to the body. Thanks to this, the electrodes 3 mounted on the vest adhere to the skin well and correctly register the heart's electrical activity. In the embodiment, the garment 2 is equipped with four electrodes 3, which are permanently attached to the belt 6 at the front of the vest. As described in the invention, the garment 2 contains four contacts 9. They are located on the front of the vest, on one of the suspenders 7. Each contact 9 is connected to one electrode 3 in a manner allowing the conduction of electrical charges. In this embodiment, contacts 9 are connected to electrodes 3 with electrical wires 8. In other embodiments, conductive thread or conductive fabrics can be used. Contacts 9 are rectangular metal strips 10, 1 cm by 1,5 cm and 2 mm thick. Four contacts 9 are placed in a rectangle. The gaps between them are two, three times the length of their sides. In other, alternative embodiments, the contacts 9 can be placed e.g. in a row or in a circle. They can also have a different shape, e.g. round. Contacts 9 can be made of stainless or acid-proof steel. This will prevent them from rusting after contact with the user's sweat.

As described in the invention, the recorder 1 is equipped with four contacts 11, which are connected to the recorder 1 printed circuit board. They are attached to the bottom wall of the recorder 1 casing. The number and placement of contacts 11 corresponds to the number and placement of contacts 9 on the garment 2. The contacts 11 are rectangular metal strips 12, 1 cm by 1,5 cm and 6 mm thick. Each metal strip 12 has a centrally located rectangular indentation 13. Each of those openings contains a magnet 14. The magnets 14 are rectangular plates 5 mm by 8 mm and 2 mm thick. In the embodiment, a neodymium magnet was used. The magnets 14 mounted in the contacts 11 attract contacts 9. This produces a detachable mount of the recorder 1 on the garment 2, allowing a fast and simple attachment and detachment. A mount of this type does not require much precision from the patient while they attach the recorder 1 to the vest worn on their body. This characteristic of the solution is particularly important when the ECG monitoring system is being used by elderly or infirm persons. In addition to a mechanical connection, contacts 9 and contacts 11 also create an electrical connection capable of sending electrical signals captured from the heart by electrodes 3 to the recorder 1. Placing magnets 14 in indentations 13, and not complete openings, screens the magnetic waves they emit, so that the waves do not cause interference which could influence the proper functioning of the recorder 1. The recorder 1 is equipped with a 32-bit processor, real-time clock, EMMC memory to save the registered signals, a power supply, 470 mAh battery and a charger circuit for charging the battery, The recorder 1 also contains an analog converter 15 to receive signal from the electrodes and convert it to digital. The analog converter 15 has three inputs 16 (C1, C2, C3) receiving signals from three electrodes 3 and one output 17 (N) sending signals to the fourth electrode 3. The inputs 16 and output 17 are connected by an analog to digital converter 18. The inputs 16 C1, C2, C3 have 9 kHz low-pass filters 19. The output 17 N has a 28 kHz low-pass filter 20. Additionally, a buffer 21 is installed between the analog to digital converter 18 and output 17 N. The recorder 1 is also equipped with a circuit 22 switching the recorder's work mode from electrode 3 signal recording mode to charging mode and transmitting data to the docking station 4. The recorder 1 work mode switch 22 contains a set of four reed switches 23 installed in the recorder 1, each of which is assigned to a different contact 11. Switching the reed switches 23 causes the recorder 1 to switch work modes from saving data from electrodes 3 mode to sending data to the docking station 4 mode, and to charging the recorder 1 battery mode. When the reed switches 23 are within a magnetic field, they switch, which in turn sends a signal to change the recorder 1 work mode. The reed switches 23 are placed near the bottom wall of the recorder 1 casing for easier access to the magnetic field which will cause them to switch. As described in the invention, the source of the magnetic field switching the reed switches are two magnets 24 installed in the docking station 4. Placing the recorder 1 on the top wall of the docking station 4 will place the reed switches 23 in the magnetic field of the magnets 24. Thanks to two magnets 24 being used, regardless of the direction in which the recorder 1 was placed, the reed switches 23 will always be within the field of one of the magnets. The recorder is equipped with a diode bridge, so data migration and charging is possible regardless of the direction in which the recorder 1 was placed on the docking station 4. As described in the invention, the recorder 1 is equipped with a user interface consisting of a button 25, an RGB diode 26 and a buzzer 27. The interface allows the user to interact with the recorder 1. The button 25 switches the device on and off. The RGB diode 26 informs of device status, depending on its colour. The buzzer 27 alerts to sudden events, e.g. loss of connection between contacts 11 and contacts 9.

As described in the invention, the docking station 4 downloads data from the recorder 1 and sends it to a server 5 for analysis. Another task of the docking station is to charge the recorder 1. The docking station 4 is a freestanding device connected to an external power source. To send data via internet or GSM network, the docking station 4 is equipped with a router, a 3G modem and an internet port. The docking station 4 has a set of four contacts 28 placed directly under the upper wall of the docking station 4 casing. The placement of contacts 28 corresponds with the placement of contacts 11. Each of the contacts 28 is a metal strip 29 with a pogo pin 30. The metal strips 29 have the same dimensions as metal strips 10. The placement of the strips 29 under the upper wall of the docking station 4 wall causes them to be at a certain distance from the magnets 14 when the recorder 1 is placed on it. When the recorder 1 is placed on the docking station 4 horizontally, it does not need to be attached securely. This is why a weaker magnetic influence on the docking station 4 contacts is advisable. The pogo pins 30 create the electrical connection between contacts 28 and contacts 11. When the recorder 1 is placed on the docking station 4, the magnets 24 switch the reed switches 23, starting data transmission to the docking station 4 and, simultaneously, charging the recorder 1 battery. To charge the recorder 1 battery, the docking station is equipped with a recorder 1 charging circuit 31. The docking station is also equipped with a power switch 32, containing a reed switch. When the reed switch 33 is outside of any magnetic field, the power switch 32 cuts off the electricity flowing to the contacts 28. Conversely, when the reed switch 33 is switched by the influence of a magnetic field, the power switch 32 is activated and electricity flows to the contacts 28. To switch the reed switch 33 and activate the power switches 32, the recorder 1 has a magnet installed. The magnet is placed near the upper wall of the recorder 1. The docking station 4 is equipped with flash memory and a USB port. It also has a user interface, composed of a button 34 and three diodes 35. The button 34 resets the docking station 4, while the diodes 35 communicate the device status to the user.

## Claims

1. ECG monitoring system consisting of: at least two electrodes placed in an ECG examination garment, an ECG signal recorder and a docking station, where the recorder is mounted on the garment in a detachable manner and the docking station is equipped with communication measures for sending data to a server, **characterized in that** the detachable mount of the recorder (1) to the garment (2) consists of contacts (9) placed on the garment (2) in a number corresponding to the number of electrodes (3) and contacts (11) placed on the recorder (1), whose number and placement corresponds to the number and placement of the contacts (9) and each contact (9) is connected to an electrode (3), and moreover the recorder (1) is equipped with a switch (22) changing the recorder's work mode from saving signals from electrodes (3) to sending data to the docking station (4) and charging the recorder (1).

2. The ECG monitoring system according to claim 1, **characterized in that** the contacts (9) in the garment (2) and the contacts (11) in the recorder (1) create an electrical connection between the electrodes (3) and the recorder (1).

3. The ECG monitoring system according to claim 1, **characterized in that** each contact (11) on the recorder (1) is a rectangular metal strip (12) with a central, rectangular indentation (13) with a magnet (14) installed, while the contacts (11) are attached to the bottom wall of the recorder casing (1).

4. The ECG monitoring system according to claim 1, **characterised in that** each contact (9) of the garment (2) is a rectangular metal strip (10).

5. The ECG monitoring system according to claim 1, **characterised in that** the circuit (22) switching the recorder's work mode is connected to a set of reed switches (23) placed in the recorder (1), each of which is assigned to a different contact (11).

6. The ECG monitoring system according to claim 1, **characterised in that** the recorder (1) is equipped with a user interface, consisting of a button (25), an RGB diode (26) and a buzzer (27).

7. The ECG monitoring system according to claim 1, **characterised in that** the docking station (4) is a freestanding device connected to an external power source, equipped with a set of contacts (28) corresponding to the number and placement of contacts (11) on the recorder (1), with each contact (28) being a rectangular metal strip (29) with a pogo pin (30), installed directly under the upper wall of the docking station's (4) casing.

8. The ECG monitoring system according to claim 1, **characterised in that** the docking station (4) is equipped with a charging circuit (31) the recorder (1).

9. The ECG monitoring system according to claim 1, **characterised in that** the docking station (4) is equipped with a power switch (32) containing a reed switch (33).

10. The ECG monitoring system according to claim 1, **characterised in that** the docking station (4) is equipped with a user interface consisting of a button (34) and three diodes (35).

11. The ECG monitoring system according to claim 1, **characterised in that** the garment (2) is a vest consisting of a belt (6) connected to suspenders (7).

12. The ECG monitoring system according to claim 1, **characterised in that** the electrodes (3) are mounted on the belt (6).

13. The ECG monitoring system according to claim 1, **characterised in that** the electrodes (3) are fabric electrodes.

14. The ECG monitoring system according to claim 1, **characterised in that** all the connectors (9) are connected to electrodes (3) with electrical wires (8) or conductive thread or fabric.
